# NOUVEAU FASCICULE DE BREVET EUROPEEN

(11) **EP 0 559 884 B2**
(45) Date de publication et mention de la décision concernant l'opposition: **07.04.2004**
(45) Mention de la délivrance du brevet: 03.05.2000
(21) Numéro de dépôt: 92921684.4
(22) Date de dépôt: 25.09.1992
(51) Int. Cl.: C12N 15/86, A61K 48/00, C12N 15/12, C12N 9/68

(54) **VECTEURS RECOMBINANTS VIRAUX POUR L'EXPRESSION DANS DES CELLULES MUSCULAIRES**
REKOMBINANTE VIREN VEKTOREN ZUR EXPRESSION IN MUSKELZELLEN
VIRAL RECOMBINANT VECTORS FOR EXPRESSION IN MUSCLE CELLS

(30) Priorité: 27.09.1991 FR 9111947
(43) Date de publication de la demande: 15.09.1993
(73) Titulaire: Etablissement Public dit: CENTRE NATIONAL DE LA RECHERCHE SCIENTIFIQUE (CNRS), 75007 Paris (FR)
(72) Inventeur: PERRICAUDET, Michel, F-28150 Ouarville (FR); BRIAND, Pascale, F-75015 Paris (FR); STRATFORD-PERRICAUDET, Leslie, F-28150 Ouarville (FR)
(74) Mandataire: Gutmann, Ernest
(86) Numéro de dépôt international: PCT/FR1992/000898
(87) Numéro de publication internationale: WO 1993/006223

(56) Documents cités:
- EP-A- 0 185 573
- WO-A-90/11092
- WO-A-90/13640
- WO-A-91/11525
- COLLOQUE INSERM (HUMAN GENE TRANSFER. INTERNATIONAL WORKSHOP) vol. 219, 11 Avril 1991, PARIS, FRANCE pages 271 - 272 QUANTIN, B. ET AL. 'Adenovirus as an expression vector in muscle cells application to dystrophin'
- COLLOQUE INSERM (HUMAN GENE TRANSFER, INTERNATIONAL WORKSHOP) vol. 219, 11 Avril 1991, PARIS, FRANCE pages 51 - 61 STRATFORD-PERRICAUDET, L. & PERRICAUDET, M. 'Gene transfer into animals : the promise of adenovirus'
- MEISSONNIER, E. ET AL. 'Dictionnaire des médicaments vétérinaires' 1984 , EDITIONS DU POINT VETERINAIRE , MAISON-ALFORT
- SCIENCE. vol. 252, no. 5004, 19 Avril 1991, LANCASTER, PA US pages 431 - 434 ROSENFELD, M.A. ET AL. 'Adenovirus-mediated transfer of a recombinant alpha 1-antitrypsin gene to the lung epithelium in vivo'

## Description

L'invention concerne des vecteurs recombinants d'origine virale comportant une séquence nucléotidique codant pour un polypeptide déterminé, et leur utilisation pour l'expression de ce polypeptide dans des cellules musculaires. L'invention vise également un procédé d'obtention de ces vecteurs, ainsi que leurs applications, notamment en tant que médicaments dans le domaine des pathologies musculaires.

Le problème, non résolu jusqu'à maintenant, de la diffusion directe d'un gène vers un tissu spécifique, fait obstacle au développement de la thérapie gènique dans le domaine des maladies musculaires.

Les diverses tentatives de modification du tissu musculaire réalisées jusqu'à ce jour sont principalement celle de la fusion de cellules musculaires avec un muscle hôte (Salminen, A., et al., Hum. Gene Ther. 2, 15-26 (1991); Partridge, T.A., et al., Nature 337, 176-179 (1989)), et celle procédant par injection d'ADN directement dans les muscles (Wolff, J.A. et al. Science 247, 1465-1468 (1991); Acsadi, G., New Biol. 3, 71-81 (1991)).

La méthode procédant par fusion, chez des souris, de précurseurs de cellules musculaires provenant d'un donneur normal, avec des fibres musculaires d'un hôte (Partridge, T.A., et al. cité ci-dessus) a été réalisée avec succès et cette thérapie cellulaire a fait l'objet d'essais préliminaires chez des enfants. Toutefois, cette approche semble présenter trop d'inconvénients pour être applicable au traitement de pathologies musculaires. En effet, les capacités de migration de ces précurseurs de cellules étant réduites à quelques millimètres, l'implantation cellulaire de ces derniers nécessiterait des millions d'injections pendant des heures d'anesthésie. De manière inévitable, des problèmes immunologiques, conduisant à des phénomènes de rejet, risqueraient d'apparaître, comme dans le cas de nombreuses greffes. De plus le traitement de la dystrophie musculaire de Duchenne (DMD) nécessite non seulement d'atteindre les muscles du squelette, mais également les cellules myocardiques; on imagine aisément les difficultés susceptibles d'être rencontrées pour implanter des précurseurs de cellules musculaires dans le myocarde. La thérapie cellulaire semble par conséquent peu appropriée pour le traitement de cellules malades présentant une telle dissémination dans l'organisme.

La thérapie gènique par indroduction directe in vivo d'acides nucléiques à l'intérieur d'organes, est une méthode attrayante en raison de sa simplicité, mais dont le développement se heurte à un certain nombre d'obstacles. En particulier, l'expression de gènes dans les muscles reste localisée au point d'injection (Wolff, J.A., et al. cité ci-dessus) et semble être assez limitée dans le temps, particulièrement dans le muscle cardiaque (Acsadi, G. et al., cité ci-dessus).

Le but de la présente invention est précisément de permettre l'introduction d'un très grand nombre d'acides nucléiques dans un nombre important (jusqu'à 50 % et plus) de cellules musculaires d'un organisme humain ou animal, que ces cellules musculaires soient celles des muscles du squelette ou encore celles du myocarde.

La présente invention a plus particulièrement pour but de permettre l'acheminement de ces acides nucléiques vers les cellules musculaires cibles par la circulation sanguine, tout en protégeant ces acides nucléiques de l'agression de divers constituants sanguins.

Un autre but de la présente invention est de mettre à la disposition du public des compositions pharmaceutiques permettant le traitement des maladies musculaires, et plus particulièrement des pathologies génétiques du système musculaire.

La présente invention découle de la découverte faite par les inventeurs, du fait que l'on retrouve l'activité β-galactosidaue dans de nombreux tissus après injection à des souris de vecteurs recombinants d'origine virale, plus particulièrement d'adénovirus, dans le génome desquels a été inséré le gène codant pour la β-galactosidase. Parmi ces tissus, on peut citer les poumons, le foie, l'intestin, le coeur et les muscles du squelette. L'expression du gène de la β-galactosidase est constante dans le temps, puisque la proportion des cellules de couleur bleue (coloration obtenue à la suite de l'expression de ce gène) dans le tissu musculaire est à peu près équivalente d'un mois à un autre.

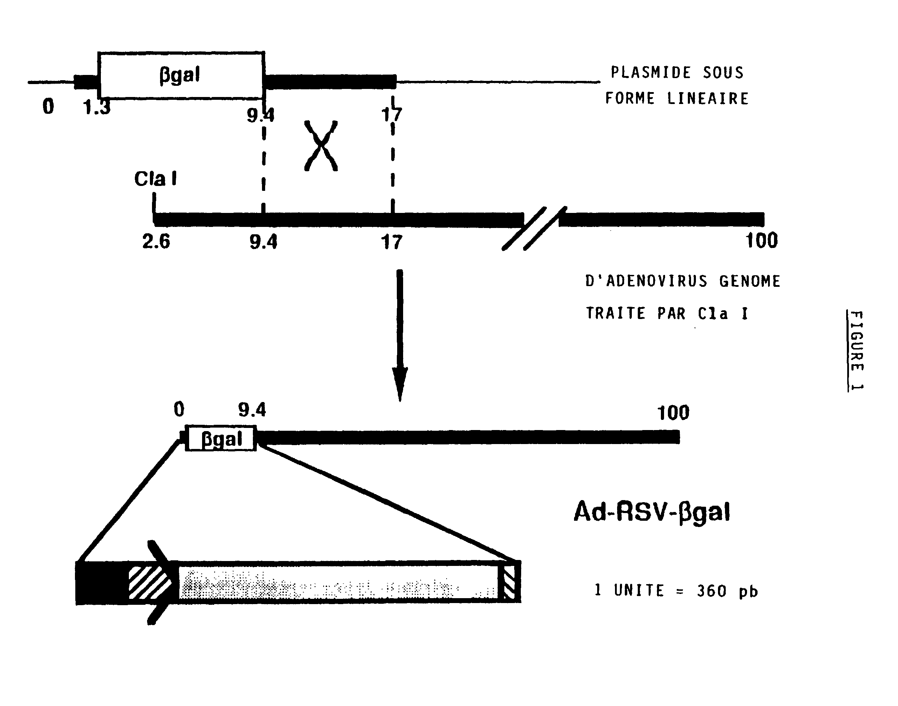

La figure 1 représente un exemple de construction d'un vecteur recombinant selon l'invention et correspondant à l'adénovirus de type Ad5 dans le génome duquel est inséré le gène de la β-galactosidase sous le controle du promoteur RSV.

La présente invention a pour objet l'utilisation de vecteurs recombinants d'origine adénovirus, non réplicables, reconnus par les récepteurs de cellules musculaires, humaines ou animales, infectables par ces virus, ces vecteurs étant en outre modifiés par un acide nucléique d'insertion contenant une séquence nucléotidique codant pour une séquence polypeptidique dont l'expression dans lesdites cellules musculaires est recherchée, cette séquence étant sous le controle d'un promoteur reconnu par les polymérases de ces cellules pour l'obtention d'un médicament administré par la voie intra-veineuse ou intra-artérielle, pour le traitement de pathologies affectant les cellules musculaires.

Les adénovirus, notamment les adénovirus humains de type 2 ou 5 représentent des vecteurs particulièrement préférés dans le cadre de la présente invention, en raison notamment de la grande taille du fragment d'ADN étranger qu'il est possible d'insérer dans le génome de ces virus.

Avantageusement, l'acide nucléique d'insertion sus-mentionné est compris dans un génome défectif d'adénovirus, ce génome étant dépourvu de séquences essentielles nécessaires à la réplication de ces adénovirus, et plus particulièrement des transactivateurs E1A et E1B; ce génome comprend néanmoins préférentiellement l'ensemble de celles des séquences essentielles nécessaires à l'encapsidation de ces adénovirus.

Le promoteur mis en oeuvre peut être un promoteur endogène (par exemple un promoteur précoce ou tardif de l'adénovirus utilisé) ou exogène.

On aura avantageusement recours à l'utilisation de promoteurs forts, par exemple ayant une force de l'ordre de grandeur du promoteur contenu dans le LTR (Long Terminal Repeat) de RSV (Rous Sarcome Virus).

A titre d'exemples d'autres promoteurs dont l'utilisation peut être envisagée, on mentionnera :
- le promoteur du gène IE de CMV (cytomégalovirus)
- les promoteurs inductibles MMTV (Mouse Mammary Tumor Virus) ou métallothionine.

La force du promoteur utilisable peut être appréciée dans des essais semblables à ceux qui sont décrits dans les exemples qui suivent, par exemple par substitution dans les vecteurs de ces exemples du promoteur étudié au promoteur contenu dans le LTR de RSV et par l'évaluation de l'intensité d'expression du marqueur obtenu, intensité qui peut alors être comparée à celle obtenue avec le promoteur de LTR de RSV.

La quantité de vecteurs administrée dans l'organisme est avantageusement choisie de manière à déborder le système immunitaire de l'organisme dans lequel ils sont injectés.

La voie d'administration choisie dans le cadre de la présente invention est la voie intra-veineuse ou intra-artérielle.

Parmi les pathologies affectant des cellules musculaires sus-mentionnées, on peut citer des pathologies génétiques telles que la dystrophie musculaire.

A ce titre l'acide nucléique inséré dans le génome du vecteur viral, et dont est recherchée la diffusion dans la masse musculaire, comprend un séquence nucléotidique codant pour un polypeptide susceptible de traiter la pathologie en question, et plus particulièrement de jouer le rôle dans la cellule musculaire du polypeptide normalement présent dans une cellule saine, mais dont la déficience est due soit à une production anormalement faible, voire nulle, de ce polypeptide, soit à une erreur dans sa séquence en acides aminés résultant d'anomalies d'ordre génétique dans sa séquence nucléotidique codante.

Des vecteurs, selon l'invention, utilisés pour l'obtention d'un médicament destiné au traitement de la dystrophie musculaire, sont plus particulièrement caractérisés en ce que l'acide nucléique d'insertion est constitué de tout ou partie d'un gène sain de la dystrophine. L'introduction du gène entier de la dystrophine, ou encore de toute partie de ce gène codant pour un polypeptide conservant une activité comparable à celle de la protéine entière, peut être réalisée suivant une méthode identique à celle décrite ci-après pour l'introduction du gène de la β-galactosidase.

A titre d'exemple de pathologies susceptibles d'être traitées dans le cadre de la présente invention, on peut citer les thromboses à l'origine des infarctus ou encore des phlébites.

Des vecteurs selon l'invention utilisés pour l'obtention d'un médicament destiné au traitement des thromboses et à la prévention des infarctus et des phlébites, sont plus particulièrement caractérisés en ce que l'acide nucléique d'insertion comprend une séquence nucléotidique codant pour une substance thrombolytique. Cette dernière séquence est avantageusement précédée d'une séquence signal codant pour un peptide, signal assurant la secrétion de la substance thrombolytique hors de la cellule musculaire.

La demande décrit également tout vecteur recombinant caractérisé en ce qu'il est constitué du génome défectif d'un adénovirus, comprenant néanmoins l'ensemble de celles des séquences essentielles nécessaires à l'encapsidation de cet adénovirus, et dans lequel est inséré un acide nucléique recombinant dont est recherchée la diffusion dans la masse musculaire, cet acide nucléique étant placé sous le controle d'un promoteur susceptible d'être reconnu par les polymérases des cellules musculaires, notamment le promoteur fort de la région précoce E1A du génome des adénovirus.

Un vecteur recombinant préféré de l'invention est caractérisé en ce que cet acide nucléique recombinant est constitué de tout ou partie du gène de la dystrophine.

Un procédé d'obtention des vecteurs recombinants est également décrit. Ce procédé comprend après l'étape de construction proprement dite de ces vecteurs par introduction de l'acide nucléique d'insertion dans leur génome, une étape de transformation de lignées cellulaires transformables d'eucaryotes supérieurs (notamment d'origine humaine ou animale) comportant elles-mêmes une séquence distincte de nucléotides apte à complémenter la partie du génome de l'adénovirus essentielle pour la réplication de ce dernier et dont le susdit vecteur est dépourvu, ladite séquence distincte étant de préférence incorporée au génome des cellules de ladite lignée cellulaire.

A titre d'exemple préféré de telles lignées cellulaires, on mentionnera la lignée 293, lignée de rein embryonnaire humain qui contient, intégrés dans son génome, les onze premiers pourcents de l'extrémité gauche du génome d'un Ad5. Ceux-ci permettent de complémenter des virus recombinants défectifs qui portent des délétions de cette région. Un tel procédé d'obtention est plus particulièrement décrit dans la demande de brevet européen n° 0185 573 du 20/11/85.

Après transformation de ces lignées cellulaires, les vecteurs qui se sont ainsi multipliés sont récupérés et purifiés.

La présente invention sera plus particulièrement illustrée à l'aide de la description détaillée qui suit de la construction d'adénovirus vecteurs recombinants comportant le gène codant pour la β-galactosidase, et des propriétés de cet adénovirus vecteur.
1. Construction de l'adénovirus recombinant, AdRSV-βgal, par recombinaison in vivo.
   Cet adénovirus recombinant a été construit par recombinaison homologue entre un plasmide approprié et le génome de l'adénovirus de type 5 (Ad5). Dans cette construction, le gène de la β-galactosidase est placé sous le contrôle du promoteur RSV (Rous Sarcoma Virus). Le plasmide pAdRSVβgal utilisé contient le segment Pvull de l'extrémité gauche de l'Ad5 (segment situé entre les positions 0 et 1,3 du plasmide sur la figure 1) comprenant la répétition terminale intervertie, l'origine de réplication, des signaux d'encapsidation, et l'amplificateur Ela. ce fragment est suivi par un gène nlslacZ (décrit dans Bonnerot, C. et al., Proc. natn. Acad. Sci. USA, 1987, 84, 6795-6799) codant pour la β-galactosidase, et par un fragment de l'adénovirus Ad5 situé entre les positions 9,4 et 17 du plasmide de la figure 1.
   Les valeurs des positions 1,3, 9,4 et 17 indiquées ci-dessus sont des unités indiquant le nombre de paires de base comprises à l'intérieur de ces fragments, une unité représentant 360 paires de base.
   La séquence d'Ad5 située entre les positions 9,4 et 17 sus-mentionnées permet la recombinaison avec l'adénovirus dl324 traité par l'enzyme de restriction Clal (correspondant à un mutant de délétion E3; la délétion étant effectuée entre les positions 78,4 et 84,3 du génome de l'adénovirus représenté sur la figure 1), après transfection de cellules 293 (cellules embryonnaires humaines de rein transformées par l'adénovirus et mentionnées ci-dessus) afin de générer le vecteur recombinant Ad-RSV-βgal. Le gène nlslacZ est contrôlé par le promoteur RSV LTR et possède le signal de polyadénylation du virus SV40. Le virus recombinant ainsi obtenu est incapable de se répliquer en raison de la délétion des gènes E1.
2. Etude du transfert du gène par l'intermédiaire de l'adénovirus aux organes de souris.
   Des souris Balb/C agées de 4 jours ont subi une injection intra-veineuse de 20-40 microlitres d'adénovirus recombinants hautement purifiés, Ad-RSV-βgal (10⁹ unités formant des plages : UFP/ml) les organes ont été prélevés 15 jours après injection et traités avec du paraformaldéhyde 4% dans un tampon phosphate pendant 30 minutes. Après rinçage les organes ont été incubés pendant une nuit à 30°C dans une solution X-gal. Les organes entiers ont ensuite été congelés et préparés de manière appropriée pour effectuer des cryosections (de 10 micromètres d'épaisseur), sections qui ont été colorées à l'aide d'hématoxyline et d'éosine.
   La mise en évidence par coloration histochimique de la manière indiquée ci-dessus de l'activité β-galactosidase sur les sections effectuées indique la présence dans les cellules des organes prélevés du gène inséré dans l'adénovirus vecteur.
   L'examen macrocospique du coeur ainsi que des muscles du squelette prélevés sur ces souris traitées, révèle la grande efficacité avec laquelle a été effectué ce transfert de gène après seulement une injection de l'adénovirus recombinant. L'intérêt du choix de la voie intraveineuse réside dans le tait que le vecteur viral n'est pas concentré dans une zone quelconque du tissu musculaire mais au contraire qu'il est favorablement dispersé dans l'ensemble de la masse musculaire. La coloration histochimique permet d'estimer que le nombre de cellules transformées atteint dans certaines zones 50 % du nombre de cellules musculaires présentes dans cette zone.
   L'expression de la β-galactosidase dans le myocarde ainsi que dans les muscles du squelette est parfaitement stable. Des colorations positives ont pu être observées 15,33,55,66,90,127 et 150 jours après l'injection de l'adénovirus recombinant. L'expression du gène semble constante en fonction du temps, puisque la proportion de cellules bleues dans les tissus musculaires semblent à peu près équivalente d'un mois à l'autre.
   L'analyse de fibres musculaires isolées révèle qu'une seule fibre est susceptible de présenter de nombreux "centres d'expression".
   Des analyses par immunotransfert (Southern) réalisées à partir du coeur d'une souris traitée ont permis de mettre en évidence une bande intense et unique correspondant à 35 Kpb indiquant que l'ADN viral introduit dans les cellules musculaires est essentiellement extrachromosomique.

## Revendications

1. Utilisation de vecteurs recombinants d'origine adénovirale, non réplicables et reconnus par les récepteurs de cellules musculaires, humaines ou animales, infectables par ces virus, ces vecteurs étant en outre modifiés par un acide nucléique d'insertion contenant une séquence nucléidique codant pour une séquence polypeptidique pour son expression dans lesdites cellules musculaires, cette séquence étant sous le contrôle d'un promoteur reconnu par les polymérases de ces cellules pour l'obtention d'un médicament administré par voie intraveineuse ou intraartérielle, pour le traitement de pathologies affectant les cellules musculaires.

2. Utilisation de vecteurs selon la revendication 1, **caractérisée en ce que** vecteurs sont choisis parmi les adénovirus défectifs dont les génomes sont dépourvus de séquences essentielles nécessaires à la réplication de ces adénovirus.

3. Utilisation selon la revendication 2, **caractérisée en ce que** le génome est dépourvu des transactivateurs E1A et E1B.

4. Utilisation de vecteurs selon l'une des revendications 1 à 3, **caractérisée en ce que** l'acide nucléique d'insertion est compris dans un génome défectif d'adénovirus comprenant néanmoins l'ensemble de celles des séquences essentielles nécessaires à l'encapsidation de ces adénovirus.

5. Utilisation de vecteurs selon l'une des revendications 1 à 4, **caractérisée en ce que** l'acide nucléique d'insertion est constitué de tout ou partie d'un gène sain de la dystrophine.

6. Utilisation de vecteurs selon l'une des revendications 1à 5, pour l'obtention d'un médicament pour le traitement de la dystrophie musculaire de Duchenne.

7. Utilisation de vecteurs selon l'une des revendications 1 à 4, pour l'obtention d'un médicament pour le traitement de maladies cardiaques, **caractérisée en ce que** l'acide nucléique d'insertion code pour une protéine ou un polypeptide ayant des propriétés thrombolytiques.

8. Utilisation selon revendication 1 pour le traitement dans les cellules de muscles du squelette.

9. Utilisation selon revendication 1 pour le traitement dans les cellules du myocarde.

## Claims

1. Use of non-replicatable recombinant vectors of adenoviral origin recognized by human or animal muscle cell receptors that can be infected by said viruses, said vectors also being modified by an insertion nucleic acid containing a nucleic acid coding for a polypeptide sequence for its expression in said muscle cells, said sequence being under the control of a promoter recognized by polymerases from said cells, for the production of a drug that is administered intravenously or intra-arterially, for the treatment of diseases affecting the muscle cells.

2. Use of vectors according to claim 1, **characterized in that** the vectors are selected from defective adenoviruses the genomes of which are free of essential sequences necessary for the replication of said adenoviruses.

3. Use according to claim 2, **characterized in that** the genome is free of E1A and E1B transactivators.

4. Use of vectors according to one of claims 1 to 3, **characterized in that** the insertion nucleic acid is comprised in a defective adenovirus genome nevertheless comprising all of the essential sequences necessary for encapsidation of said adenoviruses.

5. Use of vectors according to one of claims 1 to 4, **characterized in that** the insertion nucleic acid is wholly or partially constituted by a healthy gene for dystrophin.

6. Use of vectors according to one of claims 1 to 5, for the production of a drug for the treatment of Duchenne's muscular dystrophy.

7. Use of vectors according to one of claims 1 to 4, for the production of a drug for the treatment of cardiac disease, **characterized in that** the insertion nucleic acid encodes a protein or polypeptide having thrombolytic properties.

8. Use according to claim 1, for treatment in skeletal muscle cells.

9. Use according to claim 1, for treatment in myocardial cells.

## Patentansprüche

1. Verwendung von Rekombinations-Vektoren adenoviralen Ursprungs, die nicht replizierbar sind und von Rezeptoren menschlicher oder tierischer Muskelzellen erkannt werden, die durch diese Viren infizierbar sind, wobei diese Vektoren darüber hinaus durch eine Insertions-Nukleinsäure modifiziert sind, welche eine eine Polypeptid-Sequenz kodierende Nukleotid-Sequenz für deren Expression in den genannten Muskelzellen beinhaltet, wobei diese Sequenz unter der Kontrolle eines Promotors ist, der von Polymerasen dieser Zellen erkannt wird, zur Herstellung eines intravenös oder intraarteriös verabreichbaren Medikaments, zur Behandlung von Pathologien, die die Muskelzellen betreffen.

2. Verwendung der Vektoren nach dem Anspruch 1, **dadurch gekennzeichnet, dass** die Vektoren aus den defekten Adenoviren, deren Genome von den für die Replikation der Adenoviren notwendigen essentiellen Sequenzen befreit sind, ausgewählt werden.

3. Verwendung der Vektoren nach dem Anspruch 2, **dadurch gekennzeichnet, dass** das Genom von den Transaktivatoren E1A und E1B befreit ist.

4. Verwendung der Vektoren nach einem der Ansprüche 1 bis 3, **dadurch gekennzeichnet, dass** die Insertions-Nukleinsäure in einem defekten Genom von Adenoviren enthalten ist, das dennoch die Menge an Zellen der essentiellen Sequenzen beinhaltet, die für die Verkapselung dieser Adenoviren notwendig sind.

5. Verwendung der Vektoren nach einem der Ansprüche 1 bis 4, **dadurch gekennzeichnet, dass** die Insertions-Nukleinsäure aus dem Ganzen oder einem Teil eines gesunden Genes des Dystrophins besteht.

6. Verwendung der Vektoren nach einem der Ansprüche 1 bis 5 für die Herstellung eines Medikaments für die Behandlung der Muskeldystrophie nach Duchenne.

7. Verwendung der Vektoren nach einem der Ansprüche 1 bis 4 für die Herstellung eines Medikaments für die Behandlung von Herzkrankheiten, **dadurch gekennzeichnet, dass** die Insertions-Nukleinsäure ein Protein oder ein Polypeptid mit thrombolytischen Eigenschaften kodiert.

8. Verwendung nach Anspruch 1 für die Behandlung in Skelettmuskelzellen.

9. Verwendung nach Anspruch 1 für die Behandlung in Myocardzellen.
